# EUROPEAN PATENT APPLICATION

(11) **EP 0 630 661 A1**
(43) Date of publication of application: **28.12.1994**
(21) Application number: 93304959.5
(22) Date of filing: 24.06.1993
(51) Int. Cl.: A61M 39/02

(54) **Intravenous catheterization**

(71) Applicant: CRITIKON, INC., Tampa Florida 33634 (US)
(72) Inventor: Gomez, Geoffrey Rodriquez, Sandton, Transvaal Province (ZA)
(74) Representative: Mercer, Christopher Paul

(57) **Abstract**

An extension set 10 for use with an intravenous catheter placement unit comprising a cannula protruding from a hub, and an introducer needle protruding from a flash chamber fitted with a flash plug, with the needle extending through the cannula so that the flash chamber abuts the hub is provided. The extension set comprises a flexible conduit 12; first connecting means 50 at a first end 16 of the conduit for connecting the conduit to the cannula hub of the catheter placement unit; and second connecting means 22 at a second end 14 of the conduit for connecting the conduit to a fluid feed line of an intravenous fluid administration set. An injection port 34 for injecting a treatment substance into the conduit, is also provided.

## Description

THIS INVENTION relates to intravenous catheterization. More specifically the invention relates to an extension set for use with an intravenous catheter placement unit.

According to the invention, there is provided an extension set for use with an intravenous catheter placement unit comprising a cannula protruding from a hub, and an introducer needle protruding from a flash chamber fitted with a flash plug, with the needle extending through the cannula so that the flash chamber abuts the hub, the extension set comprising
a flexible conduit;
first connecting means at a first end of the conduit for connecting the conduit to the cannula hub of the catheter placement unit;
second connecting means at a second end of the conduit for connecting the conduit to a fluid feed line of an intravenous fluid administration set; and
an injection port for injecting a treatment substance into the conduit.

The injection port may be located at the second end of the conduit, and may be combined with the second connecting means in an injection port/second connecting means combination.

The injection port/second connecting means combination may comprise a tubular member attached to the second end of the conduit with the free end of the tubular member being connectable to the fluid feed line; a cylindrical component, for receiving a component of a syringe, protruding transversely from the tubular member, with its inside being in communication with the inside of the tubular member so that it provides the injection port; and sealing means inside the tubular member capable of permitting passage of fluid from the cylindrical component to the tubular member while preventing passage of fluid from the tubular member to the cylindrical component. The sealing means may be in the form of a sleeve of resilient material around the inside of the tubular member, with the sleeve being capable of deforming sufficiently to permit ingress of fluid from the cylindrical component to the tubular member.

The tubular member may be provided, at its free end, with outwardly protruding connecting means for connection thereto of a suitable connector on the fluid feed line. The connecting means may be lugs, a flange or the like.

The first connecting means may be a screw-threaded connector. It may be of more-or-less universal form, being connectable to the hub of different catheter placement units. It may, for example, be a so-called LUER LOCK (trade mark).

The invention will now be described by way of example with reference to the accompanying diagrammatic drawings in which:

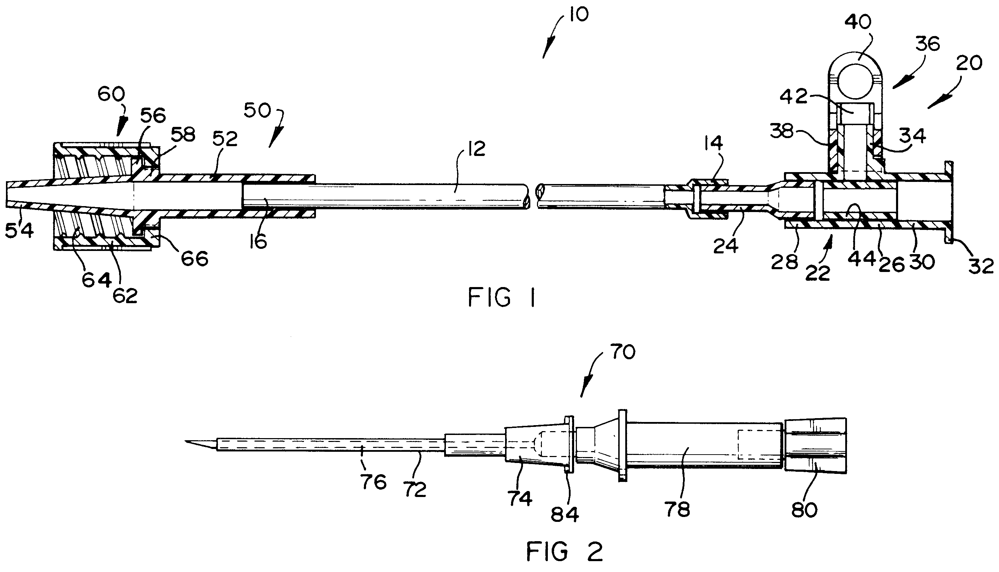

FIGURE 1 shows a longitudinal sectional view of a catheter extension set in accordance with the invention;
FIGURE 2 shows a side view of a catheter placement unit with which the catheter extension set of Figure 1 can be used; and

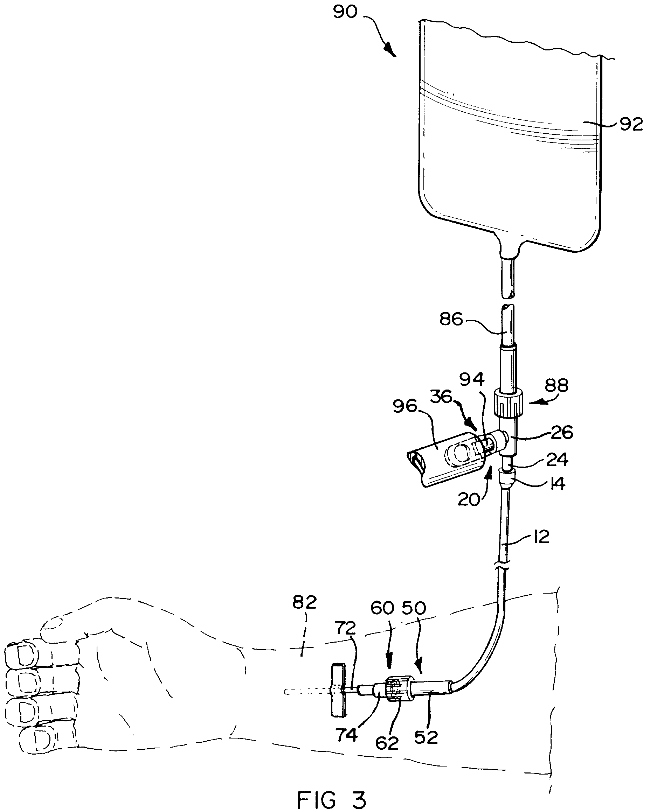

FIGURE 3 shows a three-dimensional view of the extension set of Figure 1, in use.

In the drawings, reference numeral 10 generally indicates a catheter extension set in accordance with the invention.

The extension set 10 comprises a flexible conduit or tube 12 of pliant silicone, pliant polyurethane elastomer, or the like. The tube 12 has ends 14 and 16.

To the end 14 of the tube 12 is attached a resealable injection port/connecting means combination, generally indicated by reference numeral 20. The combination 20 comprises a relatively rigid plastics tubular member, generally indicated by reference numeral 22. The tubular member 22 comprises a tube-like component 24, the one end of which fits spigot-socket fashion in the end 14 of the tube 12. The tubular member 22 also comprises a first sleeve 26, with the other end of the component 24 being located spigot-socket fashion in one end 28 of the sleeve 26. At the other end 30 of the sleeve 26 is provided a pair of diametrically opposed outwardly protruding lugs 32, the purpose of which will be described in more detail hereinafter.

The combination 20 also includes a cylindrical component 34 protruding transversely from the sleeve 26, with the interior of the component 34 being in communication with the interior or passageway of the sleeve 26. The component 34 thus provides an injection port.

To the component 34 is fitted a flip-up cap device, generally indicated by reference numeral 36. The device 36 comprises a collar 38 which fits snugly around the component 34, and a cap 40 which is connected to the collar 38 by means of a snap hinge mechanism 42. The cap 40 closes off the free end of the component 34.

The combination 20 also includes a sleeve 44 of resilient material, located snugly around the inner surface of the sleeve 26 such that it closes off the inside of the component 34 at its junction with the sleeve 26. The sleeve 44 thus provides a sealing means inside the sleeve 24 such that a fluid treatment substance, ie liquid drug or medicament, injected through the component 34 can deform the sleeve 44 sufficiently to enter the passageway of the tubular member 22. However, the sleeve 44 easily reverts to its tubular form when no fluid pressure is exerted on it from the component 34, thereby sealing against the inner surface of the sleeve 26 and preventing passage of fluid from the tubular member 22 into the component 34.

To the other end 16 of the tube 12 is fitted a connector in the form of a sliding or fixed Luer (trade mark) lock, generally indicated by reference numeral 50. The Luer lock 50 comprises a tubular portion 52 into which the tube end 16 fits spigot-socket fashion. The free or distal end 54 of the tubular portion 52 tapers down, and a radially outwardly protruding circumferential flange 56 is provided around the portion 52. A hexagonal formation 58 is provided around the portion 52, against the flange 56. Typically, the portion 52, flange 56 and formation 58 are moulded integrally from relatively rigid polymeric material. The Luer lock 50 also comprises a sleeve-like component, generally indicated by reference numeral 60. The sleeve-like component 60 comprises a collar 62, having an internal screw-thread formation 64, the purpose of which will be described in more detail hereinafter. At one end of the collar 62 is provided a radially inwardly protruding circumferential flange-like portion 66 defining an opening complementary to the hexagonal formation 58.

The extension set 10 is used with an intravenous catheter placement unit 70 comprising a cannula 72 protruding from a hub 74. The catheter placement unit 70 also comprises an introducer needle or stylet 76 extending through the cannula, and protruding from a flash chamber 78 fitted with a flash plug 80 so that the flash chamber normally abuts the hub.

In use, the cannula 72, containing the needle 76, of the assembled intravenous catheter placement unit 70 is inserted, utilizing the protruding point of the needle, into a vein in a patient's arm 82 until a flashback of blood is observed in the flash chamber 78, indicating proper insertion or entry into the vein. Thereafter, digital pressure is exerted on the vein while the needle 76 is withdrawn. The cannula 72 is inserted a desired distance into the vein, and the tapered portion 54 of the tubular portion 52 of the Luer lock 50 inserted into the cannula hub. The portion 52 is secured in position by means of the collar 62, the threads of which engage lugs 84 protruding outwardly from the cannula hub 74.

To the end 30 of the tubular member 22 is then attached a fluid feed line 86 of an intravenous administration set, generally indicated by reference numeral 90, by means of a connector 88 on the fluid feed line and which attaches over the lugs 32 protruding from the sleeve 26.

Intravenous fluid contained within a bag 92 forming part of the intravenous adminstration set 90 can then be administered to the patient via the extension set 10, in more-or-less known fashion.

When it is desired to treat the patient with a further treatment substance, such as a particular drug or medicament, the flip-up cap 40 is opened, and the substance or medicament injected through the injection port defined by the component 34, by inserting an end 94 of a syringe 96 into the free end of the component 34. The sleeve 44 then functions as a one-way valve in permitting the medicament or substance to pass into the tube 12 while preventing passage of the intravenous fluid into the component 34. Thus, a patient being treated with an intravenous fluid, can be administered a further drug or medicament at a site remote from the cannula 72 fixed to his arm. This reduces the likelihood of the cannula being disturbed and causing discomfort to the patient, or possible damage to the vein, which can arise through unnecessary movement or disturbance of the cannula 72.

A further advantage arising from the use of the extension set 10 is that the further drug or medicament is introduced directly into the stream of intravenous fluid, thus ensuring that the full dose of medicament or substance contained within the syringe is introduced into the intravenous fluid. In other words, there is no 'dead' space in which some of the medicament or drug substance being administered can temporarily remain so that it only enters the intravenous fluid at a later stage, when the patient is again administered with a drug substance or medicament. Such a drug substance remaining in a dead space or zone is undesirable, eg could lead to breakdown of the drug substance if it is light-sensitive or unstable.

Further advantages are that the set 10 can be produced at low cost, is easy to use, is of a universal nature in that it can be used with virtually any peripheral intravenous catheter placement unit or a central veinous pressure line, and it does not require the use of a hypodermic needle on the syringe for effecting injection of the drug substance, thus avoiding needle prick which could arise therefrom and associated risks involved therewith, eg infections. In view of the effective connections provided at either end of the tube 12, it can also be used for arterial catheterization.

If desired, outwardly protruding flaps or lugs can be provided on the sleeve 26 to assist in locating the extension set in position against a patient's arm or other body portion.

The tube 12 can be of any desired length, eg 5, 10 or 100 cm, and typically will have a diameter of about 4 mm.

The extension set 10 can also be used even where it is not desired to administer an intravenous fluid continually to a patient. In such case, the intravenous feed fluid line 86 can be disconnected, a suitable plug inserted into the end 30 of the sleeve 26, and a Luer lock located over the plug. In this fashion, the integrity of the tube 12 or vein patency is maintained by merely flushing it, by means of the component 34, with a suitable fluid such as heparin.

## Claims

1. An extension set for use with an intravenous catheter placement unit comprising a cannula protruding from a hub, and an introducer needle protruding from a flash chamber fitted with a flash plug, with the needle extending through the cannula so that the flash chamber abuts the hub, characterized in that the extension set comprises
a flexible conduit;
first connecting means at a first end of the conduit for connecting the conduit to the cannula hub of the catheter placement unit;
second connecting means at a second end of the conduit for connecting the conduit to a fluid feed line of an intravenous fluid administration set; and
an injection port for injecting a treatment substance into the conduit.

2. An extension set according to Claim 1, characterized in that the injection port is located at the second end of the conduit, and is combined with the second connecting means in an injection port/second connecting means combination.

3. An extension set according to Claim 2, characterized in that the injection port/second connecting means combination comprises a tubular member attached to the second end of the conduit with the free end of the tubular member being connectable to the fluid feed line; a cylindrical component, for receiving a component of a syringe, protruding transversely from the tubular member, with its inside being in communication with the inside of the tubular member so that it provides the injection port; and sealing means inside the tubular member capable of permitting fluid passage of fluid from the cylindrical component into the tubular member while preventing passage of fluid from the tubular member to the cylindrical component.

4. An extension set according to Claim 3, characterized in that the sealing means is in the form of a sleeve of resilient material around the inside of the tubular member, with the sleeve being capable of deforming sufficiently to permit ingress of fluid from the cylindrical component to the tubular member.

5. An extension set according to Claim 3 or Claim 4, characterized in that the tubular member is provided, at its free end, with outwardly protruding connecting means for connection thereto of a suitable connector on the fluid feed line.

6. An extension set according to any one of Claim 1 to 5 inclusive, characterized in that the first connecting means is a screw-threaded universal connector connectable to the hub of different catheter placement units.
